(19) Europäisches Patentamt
European Patent Office
Office européen des brevets

(11) Publication number: **0 184 307**
**A1**

(12) **EUROPEAN PATENT APPLICATION**

(21) Application number: **85307768.3**

(22) Date of filing: **28.10.85**

(51) Int. Cl.⁴: **C 01 B 35/10**
B 01 J 29/04, C 07 C 1/20

(30) Priority: **30.11.84 US 676966**
**06.11.84 US 668787**

(43) Date of publication of application:
**11.06.86 Bulletin 86/24**

(84) Designated Contracting States:
**BE DE FR GB IT NL SE**

(71) Applicant: **MOBIL OIL CORPORATION**
**150 East 42nd Street**
**New York New York 10017(US)**

(72) Inventor: **Kuehl, Guenter Hinrich**
**1956 Cardinal Lake Drive**
**Cherry Hill, N.J. 08003(US)**

(74) Representative: **Grundy, Derek George Ritchie et al,**
**CARPMAELS & RANSFORD 43, Bloomsbury Square**
**London WC1A 2RA(GB)**

(54) **Crystalline boroaluminosilicate zeolite, synthesis and use thereof.**

(57) ZSM-12 containing both aluminum and boron in its anionic framework and having the composition, on an anhydrous basis and in terms of moles of oxides:

$$(1.0 \pm 0.3)\ (a+b)\ R_{2/n}O{:}aB_2O_3{:}bAl_2O_3{:}SiO_2$$

wherein

R is at least one cation having the valence n, and a and b satisfy the relationships:

$0.0004 <= (a+b) <= 0.04$
$a > 0$, and
$b < 0.0004$.

The zeolite is synthesised from a boron-containing reaction mixture and finds application as a sorbent or as a catalyst in various organic, notably hydrocarbon, conversions.

EP 0 184 307 A1

# CRYSTALLINE BOROALUMINOSILICATE ZEOLITE, SYNTHESIS AND USE THEREOF

This invention relates to zeolite ZSM-12 containing both aluminum and boron in its anionic framework, to a method for its synthesis and to use thereof in catalytic conversion of organic compounds. ZSM-12 is generally described in US-A-3,832,449.

Crystalline boron-containing zeolites having the structure of zeolite ZSM-5 are described in US-A-4,269,813. US-A-3,941,871 discloses an organosilicate having a small amount of structurally coordinated alumina and also exhibiting an x-ray diffraction pattern similar to that of zeolite ZSM-5. US-A-3,328,119 discloses a synthetic crystalline aluminosilicate containing a minor amount of boria in its crystal framework. Other disclosures relating to various metallosilicates include US-A-3,329,480; 3,329,481 and 4,299,808. US-A-4,029,716 and 4,078,009 describe a crystalline aluminosilicate zeolite having a silica/alumina mole ratio of at least 12 and a Constraint Index within the approximate range of 1 to 12 having combined therewith boron in an amount of at least 0.2 weight percent as a result of reaction of the zeolite with a boron-containing compound. US-A-4,331,641 discloses a method for preparing a crystalline boron-containing silicate having the structure of zeolite ZSM-5 from a reaction mixture containing less than 100 ppm aluminum.

US-A-4,104,294 discloses an organosilicate of ZSM-12 structure having a different composition from the boroaluminosilicate of the present invention: that organosilicate contains less than 0.05 moles of alumina and no boron oxide. GB-A-2,024,790 discloses several

crystalline silica structures, though not ZSM-12, indicated to contain boron and to be devoid of any aluminum.

According to the invention, zeolite ZSM-12, containing both aluminum and boron in its anionic framework, has the composition, in terms of moles of oxides per mole of silica on an anhydrous basis:

$$(1.0\pm0.3)(a+b)R_{2/n}O:aB_2O_3:bAl_2O_3:SiO_2$$

wherein R is at least one cation having the valence n, and a and b satisfy the relationships:

$0.0004 <= (a+b) <= 0.04$,

$a > 0$, and

$b < 0.0004$.

In the synthesized form of the boroaluminosilicate, R may be an alkali or alkaline earth metal of valence n or an organic cation of a Group VA element of the Periodic Table of the Elements (Sargent-Welch Scientific Company) of valence n or a mixture thereof.

The boroaluminosilicate of the present invention exhibits unique and useful catalytic, sorptive and shape selective properties, along with a silica/alumina mole ratio of as high as 25,000 or even higher. It is known that catalytic activity and hydrophilic properties of aluminosilicates may decrease with increasing silica/alumina mole ratio. It is also known that certain aluminosilicates, e.g. Mordenite, Beta and ZSM-35, become more difficult to synthesize as the mole ratio of silica/alumina in the crystallization mixture is increased. Borosilicates (M. Taramasso et al., Proceedings of the Fifth International Conference on Zeolites, Heyden & Son Ltd., 1980, pp. 40-48) having the structure of zeolites ZSM-5, ZSM-11 and Beta can be prepared, but, like the

corresponding aluminosilicates, the hydrophilic properties thereof may be decreased by increasing the silica/boria mole ratio.

Unlike the aluminosilicate zeolites, however, the borosilicate zeolites have poor activity for acid catalyzed organic compound conversions. The boroaluminosilicate of this invention, however, overcomes the potential problems associated with high silica/alumina mole ratio aluminosilicates and with borosilicates in that it exhibits unique catalytic activity and hydrophilic properties, distinguishing it from known aluminosilicates and borosilicates.

The original alkali or alkaline earth metal cations of the as synthesized boroaluminosilciate can be replaced in accordance with techniques well known in the art, at least in part, by ion exchange with other cations. Preferred replacing cations include metal ions, hydrogen ions, hydrogen precursor, e.g., ammonium, ions and mixtures thereof. Particularly preferred cations are those which render the boroaluminosilicate catalyticallly active, especially for hydrocarbon conversion. These include hydrogen, rare earth metal and metals of Groups IA, IIA, IIIA, IVA, IB, IIB, IIIB, IVB and VIII of the Periodic Table of the Elements.

The lattice structure of the boroaluminosilicate of this invention, containing both boron and aluminum in tetrahedrally coordinated structural positions, is that of ZSM-12.

The crystalline boroaluminosilicate of the present invention can be thermally treated, either before or after ion exchange, by heating in an atmosphere such as air, nitrogen, hydrogen, steam, etc., at a temperature of from about 370°C to about 110°C, preferably from about 370°C to about 750°C, for from about 1 minute to about 20

hours. While subatmospheric or superatmospheric pressures may be used for this thermal treatment, atmospheric pressure is desired for reasons of convenience.

Zeolite ZSM-12 is defined by its x-ray diffraction pattern which distinguishes it from other zeolites. The x-ray diffraction pattern of the boroaluminosilicate of the present invention, having the structure of zeolite ZSM-12, is thus as set forth in the following Table:

TABLE 1

| Interplanar d-Spacing (Å) | Relative Intensity, I/Io |
|---|---|
| 11.9 ± 0.2 | M |
| 10.0 ± 0.2 | W-M |
| 6.01 ± 0.1 | W |
| 5.82 ± 0.1 | W |
| 4.73 ± 0.1 | W |
| 4.63 ± 0.1 | W |
| 4.43 ± 0.08 | W |
| 4.23 ± 0.08 | VS |
| 4.06 ± 0.08 | W |
| 3.955 ± 0.08 | W |
| 3.87 ± 0.07 | S |
| 3.82 ± 0.07 | M-S |
| 3.53 ± 0.07 | W |
| 3.44 ± 0.07 | M |
| 3.38 ± 0.07 | W |
| 3.31 ± 0.07 | W |
| 3.18 ± 0.06 | W |
| 3.04 ± 0.05 | W |
| 2.88 ± 0.05 | W |
| 2.65 ± 0.04 | W |
| 2.51 ± 0.03 | M |
| 2.43 ± 0.03 | W |
| 2.33 ± 0.02 | W |
| 2.03 ± 0.02 | W |
| 1.93 ± 0.02 | W |

These x-ray diffraction data were collected with the Philips APD-3600 x-ray system, using copper K-alpha radiation. The positions of the peaks, expressed in degrees 2 theta, where theta is the Bragg angle, were determined by step-scanning at 0.02 degrees of 2 theta intervals and a counting time of 2 seconds for each step. The interplanar spacings, d, measured in Angstrom units (A), and the relative intensities of the lines $I/I_o$, where $I_o$ is one-hundredth of the intensity of the strongest line, including subtraction of the background, were derived with the use of the software "ADP Peak Algorithm". The relative intensities are given in terms of the symbols vs = very strong, s = strong, m = medium and w = weak. It should be understood that this x-ray diffraction pattern is characteristic of all the species of zeolite compositions synthesized by the present invention. Ion exchange of the alkali metal cations with other ions results in a zeolite which reveals substantially the same x-ray diffraction pattern with some minor shifts in interplanar spacing and variation in relative intensity. Other minor variations can occur, depending on the silica to alumina plus boron oxide ratio and the boron to aluminum plus boron ratio of the particular sample, as well as its degree of thermal treatment.

Zeolite ZSM-12 exhibits an important characteristic of crystal structure of constrained access to, and egress from the intracrystalline free space by virtue of having a pore dimension greater than about 5 Angstroms and pore windows of about a size such as would be provided by distorted 12-membered rings of oxygen atoms. It is to be understood, of course, that these rings are those formed by the regular disposition of the tetrahedra making up the anionic framework of the crystalline zeolite, the oxygen atoms themselves being bonded to the silicon or

aluminum or boron atoms at the centers of the tetrahedra. The crystalline boroaluminosilicate of the invention freely sorbs normal hexane and cyclohexane, but provides constrained access to some larger molecules. It is sometimes possible to judge from a known crystal structure whether such constrained access exists. For example, if the only pore windows in a crystal are formed by 8-membered rings of oxygen atoms, then access by molecules of larger cross-section than normal hexane or cyclohexane is substantially excluded and the zeolite is not of the present type. Zeolites with windows of distorted 12-member rings are preferred for certain reactions, although excessive puckering or pore blockage may render these zeolite substantially ineffective.

Rather than attempt to judge from crystal structure whether or not a zeolite possesses the constrained access, a simple determination of the "Constraint Index" may be made by continuously passing a mixture of equal weight of normal hexane and 3-methylpentane over a small sample, approximately 1 gram or less, of zeolite at atmospheric pressure according to the following procedure. A sample of the zeolite, in the form of pellets or extrudate, is crushed to a particle size about that of coarse sand and mounted in a glass tube. Prior to testing, the zeolite is treated with a stream of air at 1000°F for at least 15 minutes. The zeolite is then flushed with helium and the temperature adjusted between 550°F and 950°F to give an overall conversion between 10% and 60%. The mixture of hydrocarbons is passed at 1 liquid hourly space velocity (i.e., 1 volume of liquid hydrocarbon per volume of catalyst per hour) over the zeolite with a helium dilution to give a helium to total hydrocarbon mole ratio of 4:1. After 20 minutes on Stream, a sample of the effluent is taken and analyzed, most conveniently by gas

chromatography, to determine the fraction remaining unchanged for each of the two hydrocarbons.

The "Constraint Index" is calculated as follows:

$$\text{Constraint Index} = \frac{\log_{10}\text{(fraction of n-hexane remaining)}}{\log_{10}\text{(fraction of 3-methylpentane remaining)}}$$

The Constraint Index approximates the ratio of the cracking rate constants for the two hydrocarbons and is 2 at 600°F for aluminosilicate zeolite ZSM-12, and from about 1 to about 4 for the boroaluminosilicate ZSM-12 of the present invention in the above temperature range of 550°F to 950°F.

The crystal framework density in the dry hydrogen form of aluminosilicate ZSM-12 is about 1.8 g/cc, and from about 1.75 g/cc to about 1.85 g/cc for the boroaluminosilicate ZSM-12 of the present invention.

The as-synthesized crystalline boroaluminosilicate of this invention exhibits an informative MASNMR (Magic Angle Spinning Nuclear Magnetic Resonance) spectrum. The techniques employed in obtaining such MASNMR spectra are described by C.A. Fyfe et al. in J. Phys. Chem. 1982, 86, 1247-1250. Under appropriate experimental conditions the $^{27}Al$ spectra show absorption (also referred to as "chemical shift") from about +50 ppm to about +60 ppm with respect to an aqueous aluminum chloride ($AlCl_3$) external standard, indicative of tetrahedral aluminum coordination in the anionic framework, and the $^{11}B$ spectra show absorption ("chemical shift") from aobut -3.5 ppm to about -6.5 ppm with respect to a borontrifluoride etherate (BTE) external standard, indicative of tetrahedral boron coordination in the anionic framework. As expected, the intensities of the $^{11}B$ and

$^{27}Al$ absorptions increase as the boron and aluminum contents of the boroaluminosilicates increase.

In general, the boroaluminosilicate of the present invention can be prepared from a reaction mixture containing a source of cations, such as, for example, organic nitrogen-containing cations, an alkali or alkaline earth metal ion source, a source of silicon such as, for example, a silicate, a source of aluminum such as, for example, an aluminate, water and a source of boron such as, for example, an oxide of boron, e.g. a borate or boric acid. The reaction mixture will have a composition, in terms of mole ratios of oxides, within the following ranges with neutralization by boric acid, when present, disregarded:

| | Broad | Preferred |
|---|---|---|
| $OH^-/SiO_2$ | 0.3-0.8 | 0.3-0.6 |
| $H_2O/OH^-$ | 60-500 | 80-300 |
| $SiO_2/Al_2O_3$ | greater than 5,000 | 20,000-200,000 |
| $SiO_2/B_2O_3$ | 5-500 | 8-300 |
| R'/R'+M | 0.30-0.90 | 0.40-0.80 |

wherein R' represents organic cations and M represents alkali or alkaline earth metal ions.

Reaction conditions consist of heating the foregoing reaction mixture to a temperature of from about 100°C to about 200°C for a period of time of from about 48 hours to about 100 days. A more preferred temperature range is from about 140°C to about 180°C with the amount of time at a temperature in such range being from about 100 hours to about 50 days.

The digestion of the gel particles is carried out until crystals of the desired boroaluminosilicate form. The crystalline product is recovered by separating it from the reaction medium, as by cooling the whole to room temperature, filtering and washing at conditions including

a pH above 7.

The above reaction mixture composition can be prepared utilizing materials which supply the appropriate oxides. Such compositions may include sodium silicate, silica hydrosol, silica gel, silicic acid, sodium hydroxide, a source of aluminum, a source of boron and an appropriate organic compound. The source of aluminum may be an added aluminum-containing compound or silica-containing materials or alkali metal-containing materials containing aluminum. The source of boron may be an oxide of boron such as borate or boric acid. The organic compound acts as a directing agent and contains an element of Group VA, such as nitrogen or phosphorus. Tetraethylammonium cation sources may be used to direct synthesis of boroaluminosilicate having the structure of ZSM-12 under appropriate conditions. These, in fact, are prescribed cation sources for aluminosilicate ZSM-12 in US-A-3,832,449: however, it has also been found that the boroaluminosilicate of the present invention can also be directed by a source of methyltriethylammonium cations.

In selecting the reaction mixture materials, care must be taken to select substantially pure sources of silicon, aluminum and boron to insure the relatively low level of aluminum in the crystal lattice of the product. For example, if a source of silicon containing about 0.5% aluminum oxide (based on solids) is used, the product crystals will contain about 0.5% or more alumina, more than desired of the present boroaluminosilicate. Preferred silicon sources include fume silica (e.g. Cab-O-Sil, (having only 20 ppm alumina per 100 grams of $SiO_2$) and tetraethylorthosilicate (having about 20 ppm alumina). Likewise, aluminum sources such as a sodium aluminate and $Al(NO_3)_3$ are preferred.

Another way to direct synthesis of the present

boroaluminosilicate molecular sieve having the crystal structure of ZSM-12 is to provide seed crystals of the boroaluminosilicate zeolite in the reaction mixture initially, suitably in the proportion of about 0.01 percent, preferably at least about 0.1 percent and still more preferably at least about 1 percent seed based on total reaction mixture weight.

Catalysts comprising a boroaluminosilicate according to the invention can be shaped into a wide variety of particle sizes. Generally speaking, they can be in the form of a powder, a granule, or a molded product, such as an extrudate having particle size sufficient to pass through a 2 mesh (Tyler) screen and be retained on a 400 mesh (Tyler) screen. In cases where the catalyst is molded, such as by extrusion, the zeolite can be extruded before drying or partially dried and then extruded.

It may be desired to incorporate the new boroaluminosilicate crystal with another material resistant to the temperatures and other conditions employed in various organic conversion processes. Such materials include active and inactive material and synthetic or naturally occurring zeolites as well as inorganic materials such as clays, silica and/or metal oxides, e.g. alumina, and are described in our EP-A-1695.

Employing a catalytically active form of the boroaluminosilicate of this invention as a catalyst component, said catalyst possibly containing additional hydrogenation components, reforming stocks can be reformed employing a temperature of from about 370°C to about 540°C, a pressure of from about 100 psig (7.9 bar) to about 1000 psig (69.95 bar), preferably from about 200 psig (14.8 bar) to about 700 psig (49.3 bar), a liquid hourly space velocity of from about 0.1 to about 10, preferably from about 0.5 to about 4, and a hydrogen to hydrocarbon mole

ratio of from about 1 to about 20, preferably from about 4 to about 12.

A catalyst comprising the boroaluminosilicate can also be used for hydroisomerization of normal paraffins, when provided with a hydrogenation component, e.g. platinum. Such hydroisomerization is carried out at a temperature of from about 90°C to about 375°C, preferably from about 145°C to about 290°C, with a liquid hourly space velocity of from about 0.01 to about 2, preferably from about 0.25 to about 0.50, and with a hydrogen to hydrocarbon mole ratio of from about 1:1 to about 5:1. Additionally, such a catalyst can be used for olefin or aromatic isomerization, employing a temperature of from about 200°C to about 480°C.

Such a catalyst can also be used for reducing the pour point of gas oils. This reaction is carried out at a liquid hourly space velocity of from about 10 to about 30 and at a temperature of from about 425°C to about 595°C.

Other reactions which can be accomplished employing a catalyst comprising the boroaluminosilicate containing a metal, e.g. platinum, include hydrogenation-dehydrogenation reactions and desulfurization reactions, olefin polymerization (oligomerization) and other organic compound conversions, such as the conversion of alcohols (e.g. methanol) or ethers (e.g. dimethylether) to hydrocarbons, and the alkylation of aromatics (e.g. benzene) in the presence of an alkylating agent (e.g. ethylene).

The following Examples illustrate the nature of the invention and its manner of practice. Adsorption data for comparison of sorptive capacities for water, cyclohexane and/or n-hexane were determined as follows:

A weighted sample of the calcined adsorbant was contacted with the desired pure adsorbate vapor in an

adsorption chamber, evacuated to 1 mm and contacted with 12 mm Hg of water vapor or 20 mm Hg of n-hexane or cyclohexane vapor, pressures less than the vapor-liquid equilibrium pressure of the respective adsorbate at room temperature. The pressure was kept constant (within about ± 0.5 mm) by addition of adsorbate vapor controlled by a manostat during the adsorption period, which did not exceed about 8 hours. As adsorbate was adsorbed, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant.

## EXAMPLE 1

3.2 grams of sodium hydroxide (98.1% NaOH) and 2.1 grams of boric acid were dissolved in 207.6 grams of water. To the resulting solution were added 35.5 grams of a 50% methyltriethylammonium (MTEA) chloride solution and, finally, 11.8 grams of Cab-O-Sil fume silica. The reaction mixture was aged at 50°C for 24 hours and then heated at 160°C for crystallization.

The reaction mixture had the following composition:

| | |
|---|---|
| $SiO_2/Al_2O_3$ | = 35,400 |
| $SiO_2/B_2O_3$ | = 11.2 |
| MTEA/(MTEA+Na) | = 0.60 |
| $OH^-/SiO_2$ | = 0.41 (disregarding partial neutralization by $H_3BO_3$) |
| $H_2O/OH^-$ | = 163 |

A crystalline product was obtained after 24 days at 160°C. It was filtered, washed with water and dried at

ambient temperature. The dried product gave the x-ray diffraction pattern of Table 2 below and was of small crystallite size, confirmed by electronmicroscopy.

TABLE 2

Example I - X-ray Diffraction Pattern

| Interplanar d-Spacing (Å) | Relative Intensity, I/Io |
|---|---|
| 11.94 | 27 |
| 10.10 | 12 |
| 7.35 | 1 |
| 6.04 | 2 |
| 5.83 | 3 |
| 4.73 | 13 |
| 4.64 | 10 |
| 4.43 | 5 |
| 4.23 | 100 |
| 4.06 | 8 |
| 3.84 | 56 |
| 3.72 | 7 |
| 3.63 | 5 |
| 3.52 | 10 |
| 3.44 | 25 |
| 3.38 | 18 |
| 3.31 | 16 |
| 3.18 | 8 |
| 3.05 | 8 |
| 2.881 | 9 |
| 2.803 | 2 |
| 2.717 | 2 |
| 2.643 | 3 |
| 2.573 | 2 |
| 2.503 | 21 |
| 2.427 | 6 |
| 2.375 | 1 |

| | |
|---|---|
| 2.329 | 4 |
| 2.253 | 2 |
| 2.192 | 2 |
| 2.158 | 1 |
| 2.098 | 2 |
| 2.032 | 9 |
| 1.980 | 3 |
| 1.929 | 7 |
| 1.886 | 2 |

After calcination at 550°C, the following sorptive capacities were found at ambient temperature, in g/100 g of solid:

| | |
|---|---|
| Cyclohexane, 20 Torr | 7.9 |
| n-Hexane, 20 Torr | 5.8 |
| Water, 12 Torr | 1.7 |

The composition of the boroaluminosilicate product was:

| | |
|---|---|
| $SiO_2$, wt % | 84.3 |
| $Al_2O_3$, ppm | 95 |
| $B_2O_3$, wt % | 1.71 |
| $Na_2O$, wt % | 0.30 |
| N, wt % | 0.84 |
| Ash, wt % | 87.3 |
| $SiO_2/Al_2O_3$, molar | 15,085 |
| $SiO_2/B_2O_3$, molar | 57.2 |
| B/(B+Al), atomic | 0.996 |

MASNMR analysis of the product boroaluminosilicate of this Example indicates a $^{11}B$ chemical shift of from -3.5 ppm to -6.5 ppm relative to borontrifluoride etherate external standard and a $^{27}Al$ chemical shift of from +50 ppm to +60 ppm relative to aqueous aluminum chloride external standard.

EXAMPLE 2

A dried sample of the Example 1 product was sized 14-25 mesh and calcined in a tube furnace in flowing nitrogen for 3 hours at 538°C, using 2 hours to increase the temperature to this level. Any carbon deposited in the boroaluminosilicate by this procedure was subsequently burnt off in flowing air. After cooling to room temperature, ammonia was sorbed.

The product was slurried with 0.2N NaCl/0.02N NaOH, 50 cc per gram of crystalline product and equilibrated for 1 hour at 71°C in a sealed polyethylene jar. The product was filtered and washed with 0.01N NaOH until the effluent was free of chloride, followed by one wash with water.

The product, dried at ambient temperature, had the following composition:

| | |
|---|---|
| $SiO_2$, wt % | 83.5 |
| $Al_2O_3$, ppm | 160 |
| $B_2O_3$, wt % | 1.61 |
| $Na_2O$, wt % | 2.0 |
| Ash, wt % | 88.1 |

The product of Example 2 gave the x-ray diffraction patterns shown in Table 3 below.

TABLE 3

Example 2 - X-ray Diffraction Pattern

| Interplanar d-Spacing (Å) | Relative Intensity, I/Io |
|---|---|
| 11.87 | 27 |
| 10.01 | 13 |
| 7.28 | 1 |
| 6.01 | 4 |
| 5.81 | 4 |
| 4.74 | 9 |
| 4.63 | 9 |

| | |
|---|---|
| 4.42 | 6 |
| 4.23 | 100 |
| 4.05 | 8 |
| 3.96 | 13 |
| 3.86 | 46 |
| 3.82 | 45 |
| 3.72 xx) | W |
| 3.63 xx) | W |
| 3.53 | 11 |
| 3.44 | 26 |
| 3.38 | 19 |
| 3.31 | 19 |
| 3.18 | 8 |
| 3.12 | 4 |
| 3.04 | 9 |
| 2.884 | 9 |
| 2.804 | 3 |
| 2.714 | 3 |
| 2.640 | 5 |
| 2.577 | 3 |
| 2.497 | 24 |
| 2.420 | 7 |
| 2.371 | 3 |
| 2.322 | 5 |
| 2.256 | 2 |
| 2.187 | 2 |
| 2.147 | 1 |
| 2.092 | 2 |
| 2.057 | 3 |
| 2.022 | 10 |
| 1.980 | 3 |
| 1.929 | 6 |
| 1.884 | 2 |

xx) Shoulder or weak line not picked up by computer.

EXAMPLE 3

The product of Example 2 was exchanged three times with 0.2N ammonium acetate solution, 45 cc per gram of zeolite, at 71°C for 2 hours each in a sealed polypropylene jar. The product was filtered and washed with copious amounts of water and dried.

The dried product had the following composition:

| | |
|---|---|
| $SiO_2$, wt % | 88.8 |
| $Al_2O_3$, ppm | 175 |
| $B_2O_3$, wt % | 1.35 |
| $Na_2O$, ppm | 54 |
| N, wt % | 0.64 |
| Ash, wt % | 91.2 |

EXAMPLE 4

Sodium hydroxide pellets, 2.0g and 2.1g of boric acid were dissolved in 140g $H_2O$. A 50% solution of methyltriethylammonium chloride, 35.5g, was blended in. Finally, a mixture of 38.2g of Ludox LS and 40g of water was added with stirring. The well-mixed reaction mixture was adjusted to pH 13.0, the mixture then was aged 24 hours at 50°C, then readjusted to pH 13.0 with NaOH. The crystallization at 160°C was complete after 124 hours. The dried product had the following composition in wt.%:

| | |
|---|---|
| $SiO_2$ (by diff.) | = 88.4 |
| $Al_2O_3$ | = 0.18 |
| $Na_2O$ | = 0.35 |
| N | = 0.99 |
| $B_2O_3$ | = 1.55 |
| Ash | = 90.5 |
| $SiO_2/(Al_2O_3+B_2O_3)$ | = 61.3 |
| $B_2O_3/(B_2O_3+Al_2O_3)$ | = 0.93 |

The crystalline product gave the x-ray diffraction pattern of ZSM-12 as shown in Table 4 and had a crystallinity of 105% compared to the reference sample.

TABLE 4

| D,A | I/Io |
|---|---|
| 11.95 | 27 |
| 11.64 | 20 |
| 10.03 | 15 |
| 7.30 | 1 |
| 6.01 | 3 |
| 5.81 | 4 |
| 4.73 | 13 |
| 4.64 | 9 |
| 4.43 | 5 |
| 4.24 | 100 |
| 4.155 | 5 |
| 4.06 | 6 |
| 3.955 | 11 |
| 3.88 | 40 |
| 3.82 | 34 |
| 3.73 | 4 |
| 3.63 | 4 |
| 3.53 | 9 |
| 3.45 | 21 |
| 3.38 | 15 |
| 3.31 | 13 |
| 3.18 | 9 |
| 3.12 | 2 |
| 3.04 | 6 |
| 2.888 | 9 |
| 2.808 | 1 |
| 2.728 | 2 |
| 2.709 | 2 |
| 2.648 | 4 |

TABLE 4

| D,A | I/Io |
|---|---|
| 2.609 | 2 |
| 2.578 | 3 |
| 2.506 | 18 |
| 2.451 | 3 |
| 2.426 | 5 |
| 2.379 | 1 |
| 2.332 | 4 |
| 2.260 | 2 |
| 2.191 | 1 |
| 2.148 | 1 |
| 2.101 | 2 |
| 2.061 | 3 |
| 2.028 | 7 |
| 1.980 | 3 |
| 1.934 | 5 |
| 1.907 | 2 |
| 1.889 | 2 |

CLAIMS

1. Zeolite ZSM-12 containing both aluminum and boron in its anionic framework and having a composition on an anhydrous basis and in terms of moles of oxides per mole of silica expressed by the formula:

$$(1.0 \pm 0.3)(a+b)\ R_{2/n}O:aB_2O_3:bAl_2O_3:SiO_2$$

wherein R is at least one cation having the valence n, and a and b satisfy the relationships:

$0.0004 <= (a+b) <= 0.04$

$a > 0$, and

$b < 0.0004$.

2. Zeolite ZSM-12 as claimed in claim 1 which has been calcined.

3. Zeolite ZSM-12 as claimed in claim 1 or claim 2 which has been base-exchanged with hydrogen, hydrogen-precursor, rare-earth and/or Groups IA, IIA, IIIA, IVA, IB, IIB, IIIB, IVB and VIII cations.

4. Zeolite ZSM-12 according to any preceding claim in which b is less than 0.00004.

5. A method for synthesizing the zeolite claimed in claim 1 which comprises preparing a reaction mixture containing a source of organic cations, a source of silicon, a source of aluminum, a source of boron, a source of alkali or alkaline earth metal ions and water, and having the composition, in terms of moles of oxides:

| | |
|---|---|
| $OH^-/SiO_2$ | 0.3-0.8 |
| $H_2O/OH^-$ | 60-500 |
| $SiO_2/Al_2O_3$ | greater than 5,000 |
| $SiO_2/B_2O_3$ | 5-500 |
| $R'/R'+M$ | 0.30-0.90 |

wherein R' represents organic cations and M represents alkali or alkaline earth metal ions, maintaining said reaction mixture until the zeolite is formed, at a temperature of from about 100°C to about 200°C, and recovering the zeolite.

6. A method according to claim 5 wherein the reaction mixture has the composition, in terms of moles of oxides:

| | |
|---|---|
| $OH^-/SiO_2$ | 0.3-0.6 |
| $H_2O/OH^-$ | 80-300 |
| $SiO_2/Al_2O_3$ | 20,000-200,000 |
| $SiO_2/B_2O_3$ | 8-300 |
| $R'/R'+M$ | 0.40-0.80 |

7. A process for effecting catalytic conversion of an organic compound-containing feedstock which comprises contacting said feedstock under catalytic conversion conditions with a catalyst comprising a zeolite as claimed in any of claims 1 to 4.

8. A process according to claim 7 wherein the charge is hydrocarbon.

9. A process according to claim 7 or claim 8 wherein the conversion is reforming, paraffin hydroisomerisation, olefin or aromatic isomerisation, dewaxing, polymerisation, hydrodenitrogenation, hydrodesulphurisation or aromatic alkylation.

10. A process according to claim 7 wherein the conversion is of alcohols or ethers to hydrocarbons.

0184307

European Patent Office

# EUROPEAN SEARCH REPORT

Application number
EP 85 30 7768

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| A | EP-A-0 059 059 (ICI)<br>* Example 8 *<br>--- | | C 01 B 35/10<br>B 01 J 29/04<br>C 07 C 1/20 |
| A | EP-A-0 046 504 (BASF)<br>* Examples 3,1 *<br>----- | | |
| | | | TECHNICAL FIELDS SEARCHED (Int. Cl.4) |
| | | | C 01 B 35/00 |

The present search report has been drawn up for all claims

| Place of search | Date of completion of the search | Examiner |
|---|---|---|
| THE HAGUE | 18-03-1986 | BREBION J.CH. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82